# EUROPEAN PATENT SPECIFICATION

(11) **EP 3 002 084 B1**
(45) Date of publication and mention of the grant of the patent: **24.04.2019**
(21) Application number: 15185613.5
(22) Date of filing: 17.09.2015
(51) Int. Cl.: B25C 1/00, A61B 17/064, A61C 8/00, A61B 17/068

(54) **DEVICE FOR GENERATING AN IMPULSIVE FORCE AND NAIL GUN THEREFOR**
VORRICHTUNG ZUR ERZEUGUNG EINER IMPULSKRAFT UND NAGELPISTOLE DAFÜR
DISPOSITIF PERMETTANT DE GÉNÉRER UNE FORCE D'IMPULSION ET PISTOLET À CLOUER ASSOCIÉ

(30) Priority: 30.09.2014 IT MO20140276
(43) Date of publication of application: 06.04.2016
(73) Proprietor: C.G.M. S.P.A., 42015 Correggio (Reggio Emilia) (IT)
(72) Inventor: PARMIGIANI, Corrado Saverio, 42015 CORREGGIO (REGGIO EMILIA) (IT)
(74) Representative: Paparo, Aldo

(56) References cited:
- JP-A- S62 137 046
- US-A- 2 065 659
- US-A1- 2002 121 539
- US-A1- 2012 172 885

## Description

The present invention has for an object a device for generating an impulsive force and nail gun thereof.

The invention is particularly destined to a device for generating an impulsive force and related nail gun for inserting a micro-nail into a bone tissue such as a gingival tissue, for example for applications of bone implantology in the field of dentistry.

Within the dentistry profession, the micro-nail or nail, is a very small nail which is specifically used for securing membranes, osteosynthesis plates or any other "mechanical" supports grafted on the patient's gingiva in order to favour bone and tissue regeneration, for example in the case of a dental implant.

The term "nail" is used in the specific jargon within the technical scope of the present invention to define an object, preferably a metal object, exhibiting a shaped head, a stem and a sharp end which is so conformed as to be inserted into a bone tissue. Needless to say that a nail used for bone implant purposes in the dentistry field is much smaller than a conventional nail, by way of example, it exhibits a diameter of a few tenths of a millimeter and a stem length of a few millimeters.

Application of the nail into the bone tissue generally occurs by means of a device suitable for generating an impulsive force of such intensity to overcome the resistance provided by the gingival bone tissue and to cause the nail to penetrate until a pre-determined position is reached. For this reason, even micro nails as well as common nails destined for surgical use, exhibit a head which is suitable to receive the impulsive force for application thereof to the tissue.

In the prior art, the impulsive force needed for the nail to be inserted into the gingival bone tissue, is generated manually by a surgeon with the aid of a pair of tools, of which the first one consists of a small hammer producing the impulsive force, whilst the second one is suitable for keeping in position the nail and transmitting the impulsive force to the nail until the latter consolidates in the bone tissue.

The tool used for holding the nail is termed in jargon nails positioner. In particular, the nails positioner exhibits a spindle shape at which first end the nail to be implanted is positioned and retained in a reversible manner by means of a shaped portion adapted to receive at least the head of the nail. At a second end, opposite the first, there is provided a flat head which is suitable for receiving the pulse from the small hammer by impact and to transmit it to the nail itself.

Generally, the procedure for implanting a nail into the bone tissue provides that the positioning of the nail is done manually by an experienced physician who holds it in position and repeatedly exerts an impulsive force until the nail is made to penetrate into the bone tissue.

In the prior art, the tools and methodology of which use is made for inserting a nail intended for bone implant applications, exhibit some drawbacks. Such drawbacks often make it difficult, long and sensitive the step of implanting a nail into the bone tissue.

One drawback is that almost all nail insertion operations are performed manually by the surgeon, which outcomes are therefore subject to the human factor, which aspect does not enable to attain a good repeatability of the surgical operation, but above all it does not ensure a high quality of surgical procedures and of the intervention itself. A further drawback is the rather small size of the nail and the nails positioner structure, which is bulky and therefore unsuited for properly following the positioning movements of the nail performed by the experienced physician. In particular, since the nails positioner is a stiff and rectilinear element, it may interfere with other patient's anatomical parts (i.e. lips, tongue, cheeks tissue or even the teeth), for example in the case of a surgical intervention in an area which is located in proximity of the mandibular joint, that is to say, distant from the patient's mouth.

Under such circumstances, the patient's discomfort significantly increases, who is forced to endure a painful situation and a to keep a position which is at least an uncomfortable with following increase in stress on the part of which the surgeon who is performing the implant intervention.

In a situation like the one featured above, it may happen that, once the nails positioner and the nail itself are duly arranged, such arrangement is still not enough suitable for the surgeon to be able to repeatedly transmit, in a safe and accurate manner, an impulsive force to the nails positioner and thus to the nail itself for making the latter penetrate into the patient's gingiva.

Often the unnatural position that the surgeon and/or the patient are forced to assume when a nail is being inserted, does not allow the surgeon to handle the nail positioner in an appropriate manner and to move it in the right direction, with the risk of erroneously insert the nail, thus damaging the bone tissue and causing unnecessary and further pain to the patient. A further drawback of the prior art is that during insertion of a nail, both surgeon's hands are occupied to support at least the hammer and the nail positioner, without the surgeon being therefore able to perform any further movements or actions during surgery.

An example of prior art device which only in part solves the above drawbacks is disclosed in US 2002/021539. This device is provided with means for generating an impulse, but said impulse is not effectively transmitted to the nail, such that the positioning of the nail is not satasfactory.

It is an object of the present invention to overcome one or more of the drawbacks encountered in the prior art by providing a device for generating an impulsive force and related nail gun for bone implant applications not exhibiting aforementioned drawbacks.

Therefore, the invention has the aim to provide a device for generating an impulsive force and related nail gun for bone implant applications which is able to repeatedly transmit an impulsive force to a nail in an effective manner and to maintain constant the result so obtained, possibly also along a non-rectilinear direction, between an element, whereby such impulsive force is generated, and the head of the nail to be applied.

A further object of the present invention is to provide a device for generating an impulsive force and nail gun thereof, which can be effectively used even within small or narrow spaces located between the gingiva and the cheek of the patient.

A further purpose of the disclosed device is to make it possible to maintain position of the nail and insertion thereof in a simple, repeatable and accurate manner with the aid of a nail gun which generates an impulsive force and which can be handled by the user with a single hand thus keeping the other hand free.

A further object of the present invention is to make available a device for generating an impulsive force and related nail gun for bone implant applications allowing to always position the nail correctly during insertion thereof into the bone tissue, so that physical and psychological stress on the part of the surgeon and at same time discomfort for the patient, can be relieved.

These aims and others beside are attained by a device for generating an impulsive force and related nail gun for bone implant applications according to the description of one or more of the appended claims. Further characteristics and advantages will become more apparent from the detailed description of a preferred and non-exclusive embodiment of a device for generating an impulsive force and nail gun thereof in accordance with the present invention.

Such description is illustrated in a non-limiting example in the accompanying figures, wherein:
- Figure 1 is a schematic sectional view of a device for generating an impulsive force according to the present invention;
- Figure 1A is a detail in section of a detail of Figure 1, wherein some parts illustrated in Figure 1 were removed in accordance with the present invention;

- Figure 2 is a schematic sectional view of the nail gun comprising the device of figure 1 for generating an impulsive force, in an operational use configuration thereof;
- Figure 3 is a schematic sectional view of the nail gun of Figure 2, in a further operational use configuration;
- Figure 4 is a schematic sectional view of the nail gun of Figure 2, in a further and different operational use configuration;
- Figure 4A is a detail of Figure 4 obtained by removing some parts of Figure 4 to better make visible the detail according to the present invention.

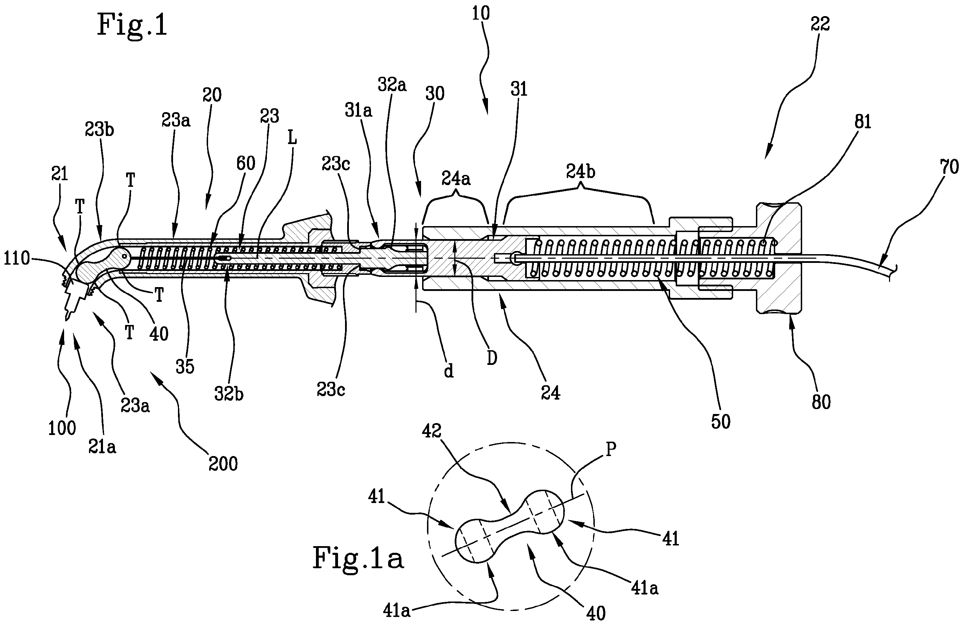

With reference to the annexed Figure 1, it was generally indicated by 10 a device for generating an impulsive force which shall be imparted to a head portion of a nail 100.

It should be noted that the term micro-nail and nail hereinafter described, are equivalent and referred to the same object, i.e. a nail 100 for bone implant applications. Additionally, the nail 100 is schematically illustrated in the appended figures in the preferred embodiment thereof, without this having to be construed as limiting the inventive concept of the present invention in any manner.

In brief, the nail 100 for bone implant applications, which further characteristics will be better illustrated hereinafter, exhibits a head portion 110 comprising a head surface, a stem and a generally sharp-shaped end portion for easier insertion thereof into the bone tissue. Between the head portion 110 and the stem, a connection portion is preferably comprised which is radiused with a pre-determined curvature radius, the head portion 110 further exhibiting a flared shape, as illustrated schematically by way of example in the appended figures.

Briefly, the device 10 for generating an impulsive force comprises a main body 20, preferably exhibiting an elongated shape, which extends from a first end 21 to a second end 22.

The main body 20 provides support and is suitable for housing the components included within the device 10 for generating a force, in a manner better described hereinafter.

It should be appreciated that the main body 20 may assume different shapes and sizes without this having to be construed as limiting the present invention in any manner, thus the schematic representation of the main body 20 in the attached Figure 1 is illustrated only by way of example.

The main body 20 has an internal cavity 23 which develops along a main line "L", which cavity 23 includes straight portions 23a and/or curved potions 23b. The above-mentioned main line "L" extends between the first end 21 and second end 22 of the main body 20. The inner cavity 23 has preferably a circular-shaped cross section.

The internal cavity 23 of the main body 20 has at least one opening 21a towards the outside 200, preferably the opening 21a is located at the first end 21. The internal cavity 23 has the main function of containing and guiding the movable elements of the device 10 for generating a force. In detail, the inner cavity 23 has at least two rectilinear portion 23a and at least one curved portion 23b interposed between the two rectilinear portions 23a. Preferably, at least one of the rectilinear portions and the curved portion 23b are arranged relative to the first end 21. Preferably, one of the two rectilinear portions 23a, particularly the one arranged at the first end 21, exhibits a shorter length compared to other rectilinear portions.

The curved portion 23b of the inner cavity 23 is such as to define an angle between the two rectilinear portions 23a preferably comprised between a maximum value of about 150 degrees and a minimum value of about 90 degrees, which angle is still more preferably comprised between a maximum value of about 120 degrees and a minimum value of about 90 degrees.

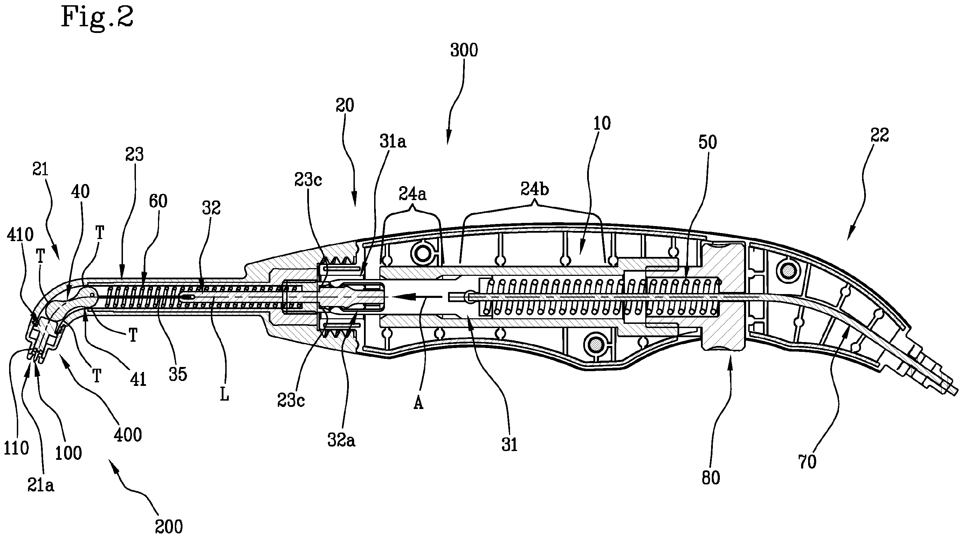

It should be appreciated that the nail 100 is disposed at the first end 21 of the main body 20. Preferably, the nail 100 is kept in position on the first end 21 by means of a head for holding a nail 400, as illustrated by way of example in the appended figures 2-4. The head for holding a nail 400 is suitable for keeping the nail 100 in position and properly guiding the nail 100 when the same is driven by the impulsive force transmitted by the abutment element 40 in an operational use configuration of the device 10 for generating a force. In other words the head for holding a nail 400 allows to operationally associate the first end 21 to the nail 100 at the opening 21a.

Further details on removal and application procedures of the nail 100 via a device 10 for generating a force, will be better illustrated hereinafter.

The device 10 for generating a force further comprises generation means 30 of the impulsive force configured for generating the impulsive force in an operational use configuration of the device 10.

Preferably, the generation means 30 is arranged relative to the second end 22 of the main body 20.

The device 10 for generating a force further comprises an abutment element 40 configured to transmit the impulsive force resulting from the generation means 30, up to a head portion 110 of a nail 100 in an operational use configuration of the device 10. However, there may be provided further elements interposed between the abutment element 40 and the head portion 110, which may be associated, by way of example, with the head for holding a nail 400.

Preferably the abutment element 40 is arranged in an intermediate position between the generation means 30 and the first end 21 of the main body 20.

In particular, the abutment element 40 is configured for sliding along the internal cavity 23, according to a stroke pulse C which is directed from the second end 22 to the first cavity 21 of the main body 20 in order to transmit the impulsive force. Furthermore, the abutment element 40 is configured for sliding along an opposite stroke B, so as to assume a reset configuration of the device 10 for generating a force.

According to a preferred but non-limiting embodiment of the present invention, the abutment element 40 comprises a bilobed-shaped body, said bilobed-shaped body particularly comprises two enlarged ends 41 between which a narrowing intermediate portion 42 is arranged relative to a reduced portion of a cross section of the bilobed-shaped body itself. Preferably, the bilobed-shaped body exhibits at least a longitudinal axis "P" and is of an elongated shape along said longitudinal axis "P", in other words the bilobed-shaped body exhibits a longitudinal dimension greater than the transverse one. Preferably, the bilobed- shaped body of the abutment element 40 is such as to be symmetrical along the longitudinal axis "P", therefore the longitudinal axis "P" is also the axis of symmetry of the abutment element 40.

The abutment element 40 is arranged within the recess 23 so that the axis of symmetry "P" is aligned or tangent to the main line "L". The enlarged ends 41 of the bilobed- shaped body have a maximum transverse dimensions such as to allow sliding of the abutment element 40 along the internal cavity 23 without mechanical interferences of any kind. Preferably, the enlarged ends 41 of the bilobed- shaped body respectively have circular-shaped cross sections, still more preferably the enlarged ends 41 have a diameter which is approximately equal to the diameter of the inner cavity 23 section.

The enlarged ends 41 of the bilobed- shaped body exhibit a measure of the circular section comprised between 2.5 mm and 6.5 mm approx., preferably between 3 mm and 5 mm approx.

Advantageously, in a sliding configuration of the bilobed-shaped body along the internal cavity 23, the bilobed-shaped body is so shaped as to have contact points "T" which lie on a surface of the inner cavity 23, which contact points "T" are grouped in two contact regions, each of which can be theoretically associated to an annular section which is comprised within a respective surface portion 41a, as shown schematically in Figure 1A hereto attached.

The surface portions 41a are cross-band shaped and include a portion of the outer surface of each enlarged end 41 relative to the region which exhibits a greater diameter than any other cross dimensions relative to the axis of symmetry "P" of the abutment element 40, in particular of the bilobed- shaped body.

In other words, two surface portions 41a are obtained on each enlarged end 41, whereon all contact points "T" of the abutment element 40 lie under a sliding condition of the latter along the internal cavity 23 of the main body 20.

According to the present embodiment, the device 10 for generating a force passes from a release condition of the impulsive force to an initial condition, wherein the impulsive force is prepared and uploaded for being delivered according to the user's needs and upon request. In this context, two operational use conditions of the device 10 for generating a force are substantially defined, wherein in one engagement condition thereof, the generation means 30 is predisposed for releasing an impulsive force for expulsion of the nail 100 and wherein, in a release condition thereof, the generation means 30 releases the impulsive force directly or indirectly onto the nail 100, thereby allowing insertion of the latter into the patient's bone tissue. The transition from the first engagement configuration to the second release configuration may occur automatically or upon user's command on the device 10.

Said generation means 30 particularly comprises engagement and release means configured for engaging with the abutment element 40, thereby dragging the same to a release position thereof, wherein said abutment element 40 releases an impulsive force for expulsion of the nail 100, under the action of elastic means. In particular, the engagement and release means comprises a first stem 31 and a second stem 32, adapted to slide along the internal cavity 23 of the main body 20. More particularly, the first stem 31 and second stem 32 are movable relative to one another in order to become mutually engaged, and subsequently integrally movable one to another in order to achieve the release condition. The first stem 31 drags the second stem 32 into the release condition, which second stem 32 in turn drags the abutment element 40 into the release condition.

According to the illustrated embodiment, the first stem 31 exhibits a pliers-shaped end 31a, for example it exhibits a shape of the type with crown of petals, which allows to reversibly receive and retain a second stem 32 via a respective rounded end 32a. Preferably, at least the pliers-shaped end 31a of the first stem 31 is flexible and deformable outwards together with the second stem 32, following interaction with a rib 32b of the second stem 32.

The second stem 32 is flexibly connected to the abutment element 40 by a flexible link 45, in particular it is connected to the bilobed-shaped body relative to an enlarged end 41, the second stem 32 is particularly connected to the enlarged proximal end 41 of the bilobed- shaped body via a respective attachment end 32b, opposite the pliers-shaped end 31a. The flexible connection 35 between the attachment end 32b of the second stem 32 and the proximal enlarged end 41 of the bilobed-shaped body may include, by way of a non-limiting example, a flexible metallic cable or a woven Kevlar® or Nylon® cable or any other flexible and non-rigid materials whatsoever.

The generation means 30 in the engagement condition thereof, are such that the second stem 32 is operationally associated to the first stem 31, preferably the first stem 31 and the second stem 32 do not move relative to one another that is, said means are mechanically connected one to another in such a way as to translate rigidly and simultaneously along the internal cavity 23. For example, such a connection is obtained owing to an interaction between the pliers-shaped end 31a of the first stem 31 and the rounded ends 32a of the second stem 32, in particular the rib 32b, as it will be described hereinafter. As shown in the attached figures, the engagement condition is obtained with the first stem 31 moving along the second end 22 towards the first end 21 (engagement stroke A of the first stem 31).

The generation means 30 in the release condition thereof, are configured to release the impulsive force directly or indirectly onto the nail 100, thereby allowing insertion thereof into a tissue by means of the abutment element 40, which abutment element 40 directly or indirectly abuts against a portion of the head 110 of nail 100 according to a pre-determined speed and force, thus conferring to the nail the force required for penetration. For example, the release is obtained by making ineffective the interaction between the pliers-shaped end 31a of the first stem 31 and the rounded end 32a of the second stem 32, particularly the rib 32b, as it will be better detailed here below. As shown in the examples attached, the release condition is obtained with an integral movement of the first stem 31 and the second stem according to the direction which goes from the first end 21 to the second end 22 (release stroke B of the first stem 31 and second stem 32). Preferably, the device 10 for generating a force exhibits the release stroke A directed opposite the direction of the engagement stroke B.

In particular, according to the present invention, the device 10 for generating a force enables to pass from the engagement configuration to the release configuration and vice versa via respective translations, or strokes of the first stem 31 and/or second stem 32 through a guide portion 24 located within the inner cavity 23.

Indeed, the guide portion 24 is configured for performing or removing the coupling which makes the first stem 31 and second stem 32 solidly constraint with one another. In particular, according to the illustrated embodiment, the guide portion 24 is configured for favouring or loosening the clamping of the pliers-shaped end 31a on the rounded end 32a of the second stem 32 via a translation of the first stem 31 and/or second stem 32, through the guide portion 24, i.e. via the engagement stroke A and the release stroke B.

The guide portion 24 preferably has a first section 24a with an internal diameter "D" approximately equal to an outer diameter "d" of the first stem 31 relative to the pliers-shaped end 31a when the latter is closed, and a second section 24b with a diameter greater than the outer diameter "d". During the engagement stroke A of the first stem 31 and the release stroke B of the first stem 31, solidly constraint with the second stem 32, the guide portion 24 enables clamping of the pliers-shaped end 31a onto the rounded end 32a of the second stem 32, such that the first stem 31 drags the second stem into the release condition thereof, and thereafter it helps the second stem 32 to be released from the first stem 31, in order to generate the impulsive force.

In detail, the first stem 31 performs the engagement stroke A towards the second stem 32 by crossing the guide portion 24 and subsequently the first stem 31 and second stem 32 perform the release stroke B by re-crossing the guide portion 24. During translation of the first stem 31 to perform the engagement stroke A with the second stem 32, the second stem 32 is slidingly constraint by means of an abutment edge 23c obtained along the internal cavity 23, as illustrated in the appended figures. When exiting the guide portion 24, the pliers-shaped end 31a encounters the rounded ends 32a and is free to flex outwardly to overcome the rib 32b. Subsequently the pliers-shaped end 31a abuts beyond the rib 32b as shown in Figure 2. The translation of the first stem 31 occurs via a pushing force which is operating on the first stem 31 and is directed from the first stem 31 towards the second stem 32, preferably along a straight section of the main line "L".

Preferably, the engagement stroke of the first stem 31 towards the second stem 32 is directed from the second end 22 of the main body 20, towards the first end 21 of the main body 20 and is generated by a first elastic element 50.

In fact, the generation means 30 of the device 10 for generating a force comprise such a first elastic element 50 which is so configured as to generate above mentioned thrust force, which thrust force is able to overcome any friction force and to put in motion the first stem 31 via the first section 24a of the guide portion 24 for performing the engagement stroke A.

By way of example, according to the present embodiment, the first elastic element 50 is a helical spring of the compression type as shown schematically in the appended figures.

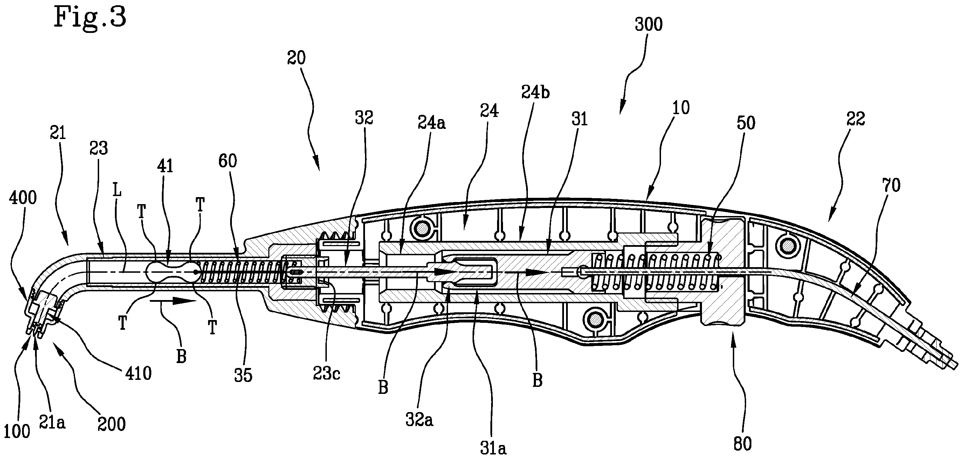

During the release stroke B, the first stem 31 and second stem 32 are engaged with one another and move integrally in the direction which goes from the first end 21 to the second end 22, by sequentially crossing the first section 24a and second section 24b of the guide portion. The guide portion 24 helps and/or maintains clamping of the pliers-shaped end 31a on the rounded end 32a of the second stem 32 by means of the first section 24a, whereas by means of the second section 24b, it enables loosening of the clamping of the pliers-shaped end 31a of the first stem 31 on the rounded end 32a of the second stem 32, thereby causing said rounded end 32a to be extracted from the pliers-shaped end 31a, thus disengaging the second stem 32 from the first stem 31, as shown schematically in the appended Figure 3.

Preferably, the first section 24a is arranged towards the first end 21 of the main body 20, while the second section 24b faces the second end 22 of the main body 20.

Therefore, during the release stroke A, the first section 24a of the guide portion 24 and thereafter the second section 24b of the guide portion 24, are initially crossed by at least the pliers-shaped end 31a of the first stem 31 (while the second stem 32 is still engaged with the first stem 31). Along the stretch going from the first section 24a, which is narrower with respect to the diameter of the guide portion 24, to the second section 24b, which is instead wider than the diameter of said guide portion 24, the stroke of the pliers-shaped end 31a allows the pliers-shaped end 31a to flex outwardly, due to the fact that the latter is usually open or subject to a recall force exerted by the second stem 32 and addressed to the first end 21, so that the rounded end 32a of the second stem 32 can be released from the first stem 31.

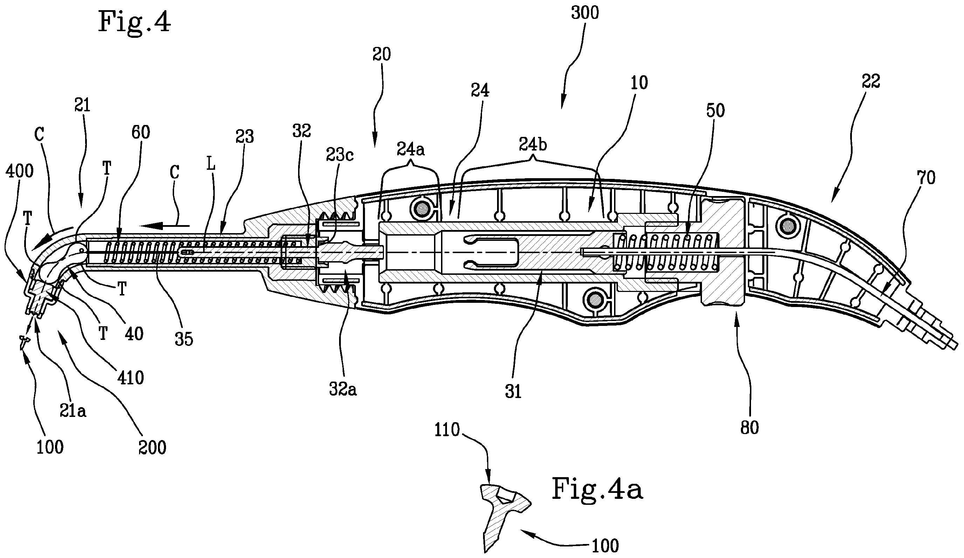

The generation means 30 particularly comprise a second elastic element 60 configured to exert above recall force, thereby generating an impulsive force onto the abutment element 40, in particular onto one of the enlarged ends 41 of the bilobed-shaped body, which impulsive force generates a pushing, thus causing said body to translate according to a pre-determined stroke pulse C (Figure 4), which is directed from the second end 22 of the main body 20 to the first end 21 of the main body 20. In particular, the second elastic element 60 is disposed between the abutment element 40 and the second stem 32 in order to keep them spaced apart from one another.

The first stem 31 in its entirety, which is engaged to the second stem 32, performs a translation or release stroke B, via the opposing force generated by the drive system 70 operated by the user, in this manner the elastic force of both the first element 50 and second element 60 is overcome simultaneously.

According to the above description, a flexible connection 35 is provided between the second stem 32 and an enlarged end 41 of the bilobed-shaped body, which flexible connection 35 is such as to allow translation of the abutment element 40 under the thrust of the second elastic element 60, and translation of the second stem 32 to the first end 21 of the main body 20.

In other words, during translation of the abutment element 40 owing to the stroke pulse C, also the second stem 32 comes closer to the first end 21 of the main body 20, which second stem 32 is dragged by the flexible connection 35 mentioned above. The stroke of the abutment element 40 along the internal cavity 23 is not limited or prevented by the flexible connection 35. The abutment edge 23c obtained along the internal cavity 23, defines the stroke limit of the second stem 32.

During the stroke pulse C, the flexible connection 35 is preferably such as to enable translation of the abutment 40, which abutment 40 thus reaches the first end 21 prior the second stem 32 having terminated its translation stroke, thereby abutting against the abutment edge 23c with a portion thereof.

In the annexed Figure 4, the nail gun 300 is illustrated schematically, wherein the stroke pulse C finally came to an end and the impulsive force was transmitted to the head portion 110 of the nail 100 by the abutment element 40 with possible interposition of an auxiliary abutment element. The nail 100 was expelled and inserted into the bone tissue, wherein axial direction, penetration depth and position were pre-established by the user. Owing to the shape of the abutment element 40, any jamming is prevented and the curved section 23b of the inner cavity 23 can be passed both during the release stroke B, but particularly during the pulse stroke C. In detail, the bilobed shape of the shutter element enables to intensify contact on the surface portions 41a before and after the narrowing portion 42.

By way of example, according to the embodiment herein described, the second elastic element 60 is a helical spring of the compression type, as shown schematically in the appended figures.

In order to fully and exhaustively understand how at least the generator means 30 of the device 10 for generating a force operate, it should be noted that said generation means 30 comprise a drive system 70 which is associated to the first stem 31 and configured for exerting an opposing force, which opposing force is greater than the thrust force exerted by the first elastic element 50 and second elastic element 60, so that the first stem 31 and second stem 32, in a condition of mutual engagement, perform the release stroke B via the guide portion 24.

According to the present embodiment, the force generating device 10 comprises an adjustment element 80 which is associated to the first elastic element 50 in such a manner that the thrust force exerted by the first elastic element 50, can be adjusted by compressing/extending the latter.

The adjustment element 80 is preferably arranged relative to the second end 22 of the main body 20 and comprises a rotatable wheel which can be actuated manually by the user and configured for being screwed into/ unscrewed from a portion of the main body 20 such as to change the position of an abutment surface 81 of the first elastic element 50 along the main line "L", in order to compress/extend the first elastic element 50 as shown in Figure 1 hereto attached.

In an alternative embodiment of the present invention, at least the generation means 30 may include electrical and electronic parts which can replace above-mentioned mechanical parts or operate therewith, with the purpose of generating a pre-determined impulsive force. By way of example, the generation means 30 may comprise an electric motor which drives a cam-shaped rotating striker so that an impulsive force is transmitted to a nail during a pre-determined step of the rotating cycle of said striker.

Alternatively, the generation means 30 may comprise pneumatic means which can replace above-mentioned mechanical parts or operate therewith, with the purpose of generating a pre-determined impulsive force.

According to the inventive concept of the present invention, it is hereinafter featured a nail gun 300 for bone implant applications, comprising a device 10 for generating an impulsive force as described above. Figures 2-4 schematically illustrate some use configurations of the nail gun 300 and which are better described hereinafter.

The nail gun 300 preferably comprises a head for holding a nail 400 suitable for bone implant interventions, still more preferably the head for holding a nail 400 is secured to the first end 21 of the main body 20 of the device 10 for generating a force.

Preferably, the head for holding a nail 400 includes a nail 100 having a head portion 110 facing towards the abutment element 40 of the device 10 for generating a force in an operating use configuration of the nail gun 300.

The head for holding a nail 400 may preferably comprise an auxiliary abutment element 410 which mainly has the function of guiding correctly the nail 100 when the same is being expelled from the head 400 and thereafter inserted into the bone tissue, thereby transmitting the impulsive force received by the abutment element 40 up to the head portion 110 of the nail 100.

The drive system 70 of the device 10 for generating a force of the nail gun 300, preferably comprises a foot lever, not illustrated in the appended figures, which is operationally associated with the actuating means 30 so that activation of the nail gun 300 through the foot is allowed; by using this part of the body, the user can in fact leave his/her hands free thus being able to better operate.

The foot lever is preferably connected by a flexible element to the opposite end 31b of the pliers-shaped end 31a of the first stem 31, so as to allow free movement and positioning of the nail gun 300 in an operational use configuration thereof. The flexible connection element located between the foot lever and the other elements of the drive system 70, can be for example a metal woven cable housed within a Teflon® protective sheath. In the appended Figures 2-4 there is illustrated schematically and by way of example, a sequence of the main use configurations of the nail gun 300 in accordance with the present invention, starting from a configuration, wherein a head for holding a nail 400 is secured at the first end 21 of the main body 20 and the nail 100 is disposed interiorly.

According to the inventive concept of the present invention, at least a portion of the main body 20 is preferably flexible at the first end 21 thereof, and orientated by the user along a pre-determined and preferred direction. Advantageously, the orientation of the first end 21 of the main body 20 and therefore also of the head for holding a nail 400, confers further flexibility of use to the nail gun 300, thereby allowing the head for holding a nail 400 and the first end 21, to be oriented in multiple position combinations. The present invention has attained the intended aim and objects, thereby overcoming above mentioned drawbacks.

Nail insertion operations by the user, are advantageously performed with a single hand, which is the one holding the nail gun as featured in the present invention.

Insertion of the nail via the inventive nail gun, advantageously allows excellent repeatability of the intervention, thus ensuring high quality surgical procedures and interventions.

The abutment element is so conformed, as to be able to advantageously run along the impulsive force and transmit the same to the nail, and to pass over one or even more curved portions of the main body of the nail gun, in particular of the end of the main body, whereon the head for holding a nail is secured and the nail is located.

Advantageously, the abutment element is so conformed as to exceed the apex of a possible curved portion of the main body without coming into contact with it, thereby allowing the impulsive force to be transmitted with a reduced dissipation, in frictional contact with the walls of the internal cavity of the main body. Unlike the prior art, such an effective transmission of the impulsive force, definitely allows the surgeon to not having to repeatedly hit the nail in the case the latter has not fully penetrated into the bone as expected.

Such a highly effective transmission of the impulsive force of the device for generating said impulsive force featured in the invention herein, advantageously allows to dispose the ends thereof, and thus the entire nail gun, in a multitude of positions, thereby enabling the surgeon to operate without any stress or fatigue.

Advantageously, owing to a high use flexibility provided by the nail gun and a highly effective insertion of the nails occurring with the aid of the nail gun herein disclosed, the patient's comfort significantly increases, who is thus no longer forced to endure a painful situation and to keep an uncomfortable position.

## Claims

1. A device (10) for generating an impulsive force, comprising:
- a main body (20) having an internal guide cavity (23) which develops along a main line (L) extending between a first end (21) and a second end (22) of said main body (20), wherein said internal cavity (23) has at least one opening (21a) towards the outside (200) at said first end (21) of said main body (20) and wherein said first end (21) is suitable for being operationally associated with a nail (100) at said opening (21a);
- generation means (30) for generating said impulsive force, said generation means (30) being configured to generate said impulsive force in an operational use configuration of said device (10), said generation means (30) being arranged at said second end (22) of said main body (20);
- an abutment element (40) configured to transmit said impulsive force produced by said generation means (30) to a portion of said nail (100) in an operational use configuration of said device (10);
wherein said internal cavity (23) has at least two straight portions (23a) and at least one curved portion (23b) comprised between said two straight portions (23a), one of said straight portions and preferably said curved portion (23b) being arranged at said first end (21) of said main body (20); wherein said generation means (30) comprises engagement and release means configured to engage the abutment element (40) and drag it to a release position in which, under the action of elastic means, it releases an impulsive force for removing the nail (100), said engagement and release means, comprising a first stem (31) and a second stem (32) suitable for sliding along the internal cavity (23) of the main body (20);
**characterized in that** said second stem (32) is flexibly connected to said abutment element (40) respectively at an attachment end (32b) of said second stem (32).

2. A device (10) according to claim 1, wherein said curved portion (23b) of said internal cavity (23) is such as to define an angle of about 90 up to 150 degrees between said straight portions (23a), the angle being preferably comprised between a maximum value of about 120 degrees and a minimum value of about 90 degrees.

3. A device (10) according to any one of the preceding claims, wherein said abutment element (40) is configured so as to slide along said internal cavity (23) according to a stroke pulse (C) directed from said second end (22) towards said first end (21) of said main body (20) for transmitting said impulsive force, said abutment element (40) being configured to slide according to an engagement stroke (B) from said first end (21) towards said second end (22) to assume a reset configuration of said device (10).

4. A device (10) according to claim 3, wherein said abutment element (40) comprises a bilobed- shaped body, said bilobed-shaped body extending along a longitudinal axis (P) and comprising two enlarged ends (41) between which an intermediate narrowing portion (42) is arranged relative to a reduced portion of the body cross section, said longitudinal axis (P) being preferably an axis of symmetry.

5. A device (10) according to claim 4, wherein said enlarged ends (41) of said bilobed- shaped body have a maximum transverse dimensions such as to allow sliding of said abutment element (40) along said internal cavity (23) without mechanical interference, preferably at least said enlarged ends (41) and said internal cavity (23) of said main body (20) having respectively circular-shaped cross sections of approximately same size.

6. A device (10) according to claim 5, wherein said bilobed- shaped body exhibits points of contact (T) arranged in contact with a surface of said internal cavity (23), said points of contact (T) being included in two surface portions (41a), each associated with an enlarged end (41), and wherein the surface portion (41a) of each enlarged end (41) has a greater diameter than any other transverse dimensions of the respective enlarged ends (41), preferably of said bilobed- shaped body.

7. A device (10) according to any one of the preceding claims 4 to 6, wherein said bilobed- shaped body exhibits a circular section of said enlarged ends (41) with a diameter of approx. 2.5 mm up to 6.5 mm, preferably comprised between approx. 3 and 5 mm.

8. A device (10) according to any one of the preceding claims, wherein said generation means (30) comprises engagement and release means, comprising a first stem (31) having a pliers-shaped end (31a) suitable for reversibly receiving and retaining a respective rounded end (32a) of a second stem (32) operationally associated with said first stem (31) in a configuration of engagement between said first stem (31) and said second stem (32), said second stem (32) and first stem (31) being slidable along said internal cavity (23) of said main body (20) and relatively movable and/or solidly constraint with one another according to the gripping and/or release condition of the pliers-shaped end (31a).

9. A device (10) according to any one of the preceding claims, wherein said internal cavity (23) has a guide portion (24) configured to promote/loosen the clamping of said pliers-shaped end (31a), preferably said guide portion (24) having a first portion (24a) with an internal diameter approximately equal to an outer diameter ("d") of said first stem (31) at said pliers-shaped end (31a) when closed, and a second portion (24b) having a greater diameter than the outer diameter ("d").

10. A device (10) according to claim 9, wherein said generation means (30) comprises a first elastic element (50) configured to generate a pushing force on said first stem (31) so that it carries out a stroke of engagement (A) through said guide portion (24) towards said second stem (32), thereby allowing engagement between said pliers-shaped end (31a) and said rounded end (32a) in an operational use configuration of said device (10), said stroke of engagement being preferably directed from said second end (22) to said first end (21) of said main body (20).

11. A device (10) according to claim 10, wherein said generation means (30) comprises a drive system (70) associated with said first stem (31) and configured to exert an opposing force exceeding said pushing force of said first elastic element (50) such that said first stem (31) and said second stem (32), in a condition of mutual engagement, perform a release stroke (B) via said guide portion (24) in an operational use configuration of said device (10), said release stroke (B) being opposite the direction of said stroke of engagement (A).

12. A device (10) according to claim 11, wherein said second portion (24b) of said guide portion (24) of said internal cavity (23) is configured in such a way as to allow the loosening of the clamping of said pliers-shaped end (31a) on said rounded end (32a) of said second stem (32) hereby enabling extraction of said rounded end (32a) from said pliers-shaped portion (31a) in an operational release condition of said engagement and release means.

13. A device (10) according to one or more of the preceding claims , wherein said generation means (30) comprises a second elastic element (60) configured to exert an impulsive force on said abutment element (40) such as to push it towards said first end (21) during a stroke pulse (C) of said means of engagement and release.

14. A device (10) according to any one of the preceding claims, wherein said pushing force of said first elastic element (50) is adjustable by means of an adjustment element (80) preferably arranged at said second end (22) of said main body (20).

15. A nail gun (300) for of bone implant applications comprising a device for generating an impulsive force (10) according to one or more of the preceding claims and a head for holding a nail (400), preferably said head for holding a nail (400) being fixed to the first end (21) of the main body (20) of said force generating device (10).

16. A nail gun (300) according to claim 15, wherein said head for holding a nail (400) includes a nail (100) having a head portion (110) directed towards said abutment element (40) of said device (10) in an operational use configuration of said nail gun (400).

## Patentansprüche

1. Vorrichtung (10) zur Erzeugung einer Impulskraft, umfassend:
- einen Hauptkörper (20), aufweisend einen internen Führungshohlraum (23), der sich entlang einer Hauptlinie (L) entwickelt, sich erstreckend zwischen einem ersten Ende (21) und einem zweiten Ende (22) des Hauptkörpers (20), wobei der interne Hohlraum (23) mindestens eine Öffnung (21a) hinführend zur Außenseite (200) am ersten Ende (21) des Hauptkörpers (20) aufweist, und wobei das zweite Ende (21) geeignet ist, um betriebswirksam mit einem Nagel (100) an der Öffnung (21a) assoziiert zu werden;
- Erzeugungsmittel (30) zum Erzeugen der Impulskraft, wobei die Erzeugungsmittel (30) ausgelegt sind, um die Impulskraft in einer Betriebsgebrauchskonfiguration der Vorrichtung (10) zu erzeugen, wobei die Erzeugungsmittel (30) am zweiten Ende (22) des Hauptkörpers (20) angeordnet sind;
- ein Anschlagselement (40), das ausgelegt ist, um die von den Erzeugungsmitteln (30) hervorgerufene Impulskraft auf einen Abschnitt des Nagels (100) in einer Betriebsgebrauchskonfiguration der Vorrichtung (10) zu übertragen,
wobei der interne Hohlraum (23) mindestens zwei gerade Abschnitte (23a) und mindestens einen gekrümmten Abschnitt (23b), eingeschlossen zwischen den zwei geraden Abschnitten (23a), aufweist, wobei einer der geraden Abschnitte und vorzugsweise der gekrümmte Abschnitt (23b) am ersten Ende (21) des Hauptkörpers (20) angeordnet sind, wobei die Erzeugungsmittel (30) Eingriffs- und Freisetzungsmittel umfassen, ausgelegt, um das Anschlagselement (40) zu greifen und es in eine Freisetzungsposition zu ziehen, in der es unter der Wirkung elastischer Mittel eine Impulskraft freisetzt, um den Nagel (100) zu entfernen, wobei die Eingriffs- und Freisetzungsmittel einen ersten Schaft (31) und einen zweiten Schaft (32) umfassen, die geeignet sind, um entlang des internen Hohlraums (23) des Hauptkörpers (20) zu gleiten,
**dadurch gekennzeichnet, dass** der zweite Schaft (32) flexibel mit dem Anschlagselement (40) jeweils an einem Anbringungsende (32b) des zweiten Schafts (32) verbunden ist.

2. Vorrichtung (10) nach Anspruch 1, wobei der gekrümmte Abschnitt (23b) des internen Hohlraums (23) ausgestaltet ist, um einen Winkel von zirka 90 bis 150 Grad zwischen den geraden Abschnitten (23a) zu bilden, wobei der Winkel vorzugsweise zwischen einem Höchstwert von zirka 120 Grad und einem Mindestwert von zirka 90 Grad umfasst ist.

3. Vorrichtung (10) nach einem der vorhergehenden Ansprüche, wobei das Anschlagselement (40) ausgelegt ist, um entlang des internen Hohlraums (23) gemäß einem Hubimpuls (C) zu gleiten, gerichtet vom zweiten Ende (22) hinführend zum ersten Ende (21) des Hauptkörpers (20), um die Impulskraft zu übertragen, wobei das Anschlagselement (40) ausgelegt ist, um gemäß einem Eingriffshub (B) vom ersten Ende (21) hinführend zum zweiten Ende (22) zu gleiten, um eine Rücksetzungskonfiguration der Vorrichtung (10) einzunehmen.

4. Vorrichtung (10) nach Anspruch 3, wobei das Anschlagselement (40) einen zweilappig geformten Körper umfasst, wobei der zweilappig geformte Körper sich entlang einer Längsachse (P) erstreckt und zwei verbreiterte Enden (41) umfasst, zwischen denen ein sich verengender Zwischenabschnitt (42) relativ zu einem reduzierten Abschnitt des Körperquerschnitts angeordnet ist, wobei die Längsachse (P) vorzugsweise eine Symmetrieachse ist.

5. Vorrichtung (10) nach Anspruch 4, wobei die verbreiterten Enden (41) des zweilappig geformten Körpers maximale Querabmessungen aufweisen, sodass das Gleiten des Anschlagselements (40) entlang des internen Hohlraums (23) ohne mechanische Interferenz erlaubt wird, wobei vorzugsweise mindestens die verbreiterten Enden (41) und der interne Hohlraum (23) des Hauptkörpers (20) jeweils kreisförmige Querschnitte ungefähr derselben Größe aufweisen.

6. Vorrichtung (10) nach Anspruch 5, wobei der zweilappig geformte Körper Kontaktpunkte (T) besitzt, die in Kontakt mit einer Oberfläche des internen Hohlraums (23) angeordnet sind, wobei die Kontaktpunkte (T) in zwei Oberflächenabschnitten (41a) eingeschlossen sind, ein jeder assoziiert mit einem verbreiterten Ende (41), und wobei der Oberflächenabschnitt (41a) eines jeden verbreiterten Endes (41) einen größeren Durchmesser als alle anderen Querabmessungen der jeweiligen verbreiterten Enden (41), vorzugsweise des zweilappig geformten Körpers, aufweist.

7. Vorrichtung (10) nach einem der vorhergehenden Ansprüche 4 bis 6, wobei der zweilappig geformte Körper einen kreisförmigen Querschnitt der verbreiterten Enden (41) mit einem Durchmesser von zirka 2,5 mm bis 6,5 mm aufweist, vorzugsweise zwischen zirka 3 und 5 mm.

8. Vorrichtung (10) nach einem der vorhergehenden Ansprüche, wobei die Erzeugungsmittel (30) Eingriffs- und Freisetzungsmittel umfassen, umfassend einen ersten Schaft (31), aufweisend ein zangenförmiges Ende (31a), geeignet, um reversibel ein jeweiliges abgerundetes Ende (32a) eines zweiten Schafts (32) aufzunehmen und zu halten, betriebswirksam verbunden mit dem ersten Schaft (31) in einer Eingriffskonfiguration zwischen dem ersten Schaft (31) und dem zweiten Schaft (32), wobei der zweite Schaft (32) und der erste Schaft (31) entlang des internen Hohlraums (23) des Hauptkörpers (20) gleiten können und relativ bewegbar und/oder fest verbunden miteinander gemäß dem Greif- und/oder Freisetzungszustand des zangenförmigen Endes (31a).

9. Vorrichtung (10) nach einem der vorhergehenden Ansprüche, wobei der interne Hohlraum (23) einen Führungsabschnitt (24) aufweist, ausgelegt, um das Festspannen des zangenförmigen Endes (31a) zu fördern/zu lockern, wobei der Führungsabschnitt (24) vorzugsweise einen ersten Abschnitt (24a) mit einem Innendurchmesser aufweist, der ungefähr gleich einem Außendurchmesser ("d") des ersten Schafts (31) am zangenförmigen Ende (31a) ist, wenn geschlossen, und einen zweiten Abschnitt (24b), aufweisend einen größeren Durchmesser als den Außendurchmesser ("d").

10. Vorrichtung (10) nach Anspruch 9, wobei die Erzeugungsmittel (30) ein erstes elastisches Element (50) umfassen, das ausgelegt ist, um eine Schubkraft auf den ersten Schaft (31) zu erzeugen, sodass dieser einen Eingriffshub (A) durch den Führungsabschnitt (24) hinführend zum zweiten Schaft (32) durchführt, wodurch das Eingreifen zwischen dem zangenförmigen Ende (31a) und dem abgerundeten Ende (32a) in einer Betriebsgebrauchskonfiguration der Vorrichtung (10) erlaubt wird, wobei der Eingriffshub vorzugsweise vom zweiten Ende (22) zum ersten Ende (21) des Hauptkörpers (20) gerichtet ist.

11. Vorrichtung (10) nach Anspruch 10, wobei die Erzeugungsmittel (30) ein Antriebssystem (70) umfassen, das mit dem ersten Schaft (31) assoziiert und ausgelegt ist, um eine entgegenwirkende Kraft auszuüben, die die Schubkraft des ersten elastischen Elements (50) übertrifft, sodass der erste Schaft (31) und der zweite Schaft (32) in einem Zustand des Ineinandergreifens einen Freisetzungshub (B) mittels des Führungsabschnitts (24) in einer Betriebsgebrauchskonfiguration der Vorrichtung (10) durchführen, wobei der Freisetzungshub (B) der Richtung des Eingriffshubs (A) entgegengesetzt ist.

12. Vorrichtung (10) nach Anspruch 11, wobei der zweite Abschnitt (24b) des Führungsabschnitts (24) des internen Hohlraums (23) ausgelegt ist, sodass das Lockern der Festspannung des zangenförmigen Endes (31a) am abgerundeten Ende (32a) des zweiten Schafts (32) erlaubt wird, wodurch das Herausziehen des abgerundeten Endes (32a) aus dem zangenförmigen Abschnitt (31a) in einer Betriebsfreisetzungskonfiguration der Eingriffs- und Freisetzungsmittel ermöglicht wird.

13. Vorrichtung (10) nach einem oder mehreren der vorhergehenden Ansprüche, wobei die Erzeugungsmittel (30) ein zweites elastisches Element (60) umfassen, das ausgelegt ist, um eine Impulskraft auf das Anschlagselement (40) auszuüben, sodass dieses hinführend zum ersten Ende (21) während eines Hubimpulses (C) der Eingriffs- und Freisetzungsmittel geschoben wird.

14. Vorrichtung (10) nach einem der vorhergehenden Ansprüche, wobei die Schubkraft des ersten elastischen Elements (50) mittels eines Einstellelements (80) einstellbar ist, das vorzugsweise am zweiten Ende (22) des Hauptkörpers (20) angeordnet ist.

15. Nagelpistole (300) für Knochenimplantatanwendungen, umfassend eine Vorrichtung zur Erzeugung einer Impulskraft (10) nach einem oder mehreren der vorhergehenden Ansprüche und einen Kopf zum Halten eines Nagels (400), wobei vorzugsweise der Kopf zum Halten eines Nagels (400) am ersten Ende (21) des Hauptkörpers (20) der krafterzeugenden Vorrichtung (10) fixiert ist.

16. Nagelpistole (300) nach Anspruch 15, wobei der Kopf zum Halten eines Nagels (400) einen Nagel (100) umfasst, aufweisend einen Kopfabschnitt (110), der hinführend zum Anschlagselement (40) der Vorrichtung (10) in einer Betriebsgebrauchskonfiguration der Nagelpistole (400) gerichtet ist.

## Revendications

1. Dispositif (10) permettant de générer une force d'impulsion, comprenant :
- un corps principal (20) comportant une cavité interne (23) de guidage se développant le long d'une ligne principale (L) se prolongeant entre une première extrémité (21) et une seconde extrémité (22) dudit corps principal (20), dans lequel ladite cavité interne (23) comporte au moins une ouverture (21a) vers l'extérieur (200) en correspondance de ladite première extrémité (21) dudit corps principal (20) et dans lequel ladite première extrémité (21) est adaptée pour être fonctionnellement associée à un clou (100) en correspondance de ladite ouverture (21a) ;
- des moyens de génération (30) servant à générer ladite force d'impulsion, lesdits moyens de génération (30) étant configurés pour générer ladite force d'impulsion dans une configuration fonctionnelle d'utilisation dudit dispositif (10), lesdits moyens de génération (30) étant disposés en correspondance de ladite seconde extrémité (22) dudit corps principal (20) ;
- un élément de butée (40) configuré pour transmettre ladite force d'impulsion produite par lesdits moyens de génération (30) à une partie dudit clou (100) dans une configuration fonctionnelle d'utilisation dudit dispositif (10) ;
dans lequel ladite cavité interne (23) comporte au moins deux parties rectilignes (23a) et au moins une partie incurvée (23b) comprise entre lesdites deux parties rectilignes (23a), une desdites parties rectilignes et de préférence ladite partie incurvée (23b) étant disposées en correspondance de ladite première extrémité (21) dudit corps principal (20) ; dans lequel lesdits moyens de génération (30) comprennent des moyens de mise en prise et de libération configurés pour se mettre en prise avec l'élément de butée (40) et pour le tirer vers une position de libération dans laquelle, sous l'action de moyens élastiques, il libère une force d'impulsion pour retirer le clou (100), lesdits moyens de mise en prise et de libération, comprenant une première tige (31) et une seconde tige (32) adaptées pour coulisser le long de la cavité interne (23) du corps principal (20) ;
**caractérisé en ce que** ladite seconde tige (32) est reliée de façon flexible au dit élément de butée (40) respectivement à une extrémité de fixation (32b) de ladite seconde tige (32).

2. Dispositif (10) selon la revendication 1, dans lequel ladite partie incurvée (23b) de ladite cavité interne (23) est telle qu'elle définit un angle d'environ 90 à 150 degrés entre lesdites parties rectilignes (23a), l'angle étant de préférence compris entre une valeur maximum d'environ 120 degrés et une valeur minimum d'environ 90 degrés.

3. Dispositif (10) selon l'une quelconque des revendications précédentes, dans lequel ledit élément de butée (40) est configuré de sorte à coulisser le long de ladite cavité interne (23) selon une course d'impulsion (C) dirigée de ladite seconde extrémité (22) vers ladite première extrémité (21) dudit corps principal (20) pour transmettre ladite force d'impulsion, ledit élément de butée (40) étant configuré pour coulisser selon une course d'engagement (B) de ladite première extrémité (21) vers ladite seconde extrémité (22) pour adopter une configuration de réinitialisation dudit dispositif (10).

4. Dispositif (10) selon la revendication 3, dans lequel ledit élément de butée (40) comprend un corps à forme bilobée, ledit corps à forme bilobée se prolongeant le long d'un axe longitudinal (P) et comprenant deux extrémités élargies (41) entre lesquelles une partie de rétrécissement (42) intermédiaire est disposée par rapport à une partie réduite de la section transversale du corps, ledit axe longitudinal (P) étant de préférence un axe de symétrie.

5. Dispositif (10) selon la revendication 4, dans lequel lesdites extrémités élargies (41) dudit corps à forme bilobée comportent des dimensions transversales maximum de manière à permettre le coulissement dudit élément de butée (40) le long de ladite cavité interne (23) sans interférence mécanique, de préférence au moins lesdites extrémités élargies (41) et ladite cavité interne (23) dudit corps principal (20) comportant respectivement des sections transversales de forme circulaire de dimension approximativement identique.

6. Dispositif (10) selon la revendication 5, dans lequel ledit corps à forme bilobée présente des points de contact (T) disposés en contact avec une surface de ladite cavité interne (23), lesdits points de contact (T) étant inclus dans deux parties de surface (41a), chacune associée à une extrémité élargie (41), et dans lequel la partie de surface (41a) de chaque extrémité élargie (41) a un diamètre supérieur à n'importe quelle autre dimension transversale des extrémités élargies (41) respectives, de préférence dudit corps à forme bilobée.

7. Dispositif (10) selon l'une quelconque des revendications précédentes de 4 à 6, dans lequel ledit corps à forme bilobée présente une section circulaire desdites extrémités élargies (41) ayant un diamètre d'environ 2,5 à 6,5 mm, de préférence compris entre environ 3 et 5 mm.

8. Dispositif (10) selon l'une quelconque des revendications précédentes, dans lequel lesdits moyens de génération (30) comprennent des moyens de mise en prise et de libération, comprenant une première tige (31) comportant une extrémité en forme de pince 31a) adaptée pour recevoir et retenir de façon réversible une extrémité arrondie (32a) respective d'une seconde tige (32) fonctionnellement associée à ladite première tige (31) dans une configuration de mise en prise entre ladite première tige (31) et ladite seconde tige (32), lesdites seconde tige (32) et première tige (31) pouvant coulisser le long de ladite cavité interne (23) dudit corps principal (20) et étant relativement mobiles et/ou solidaiment contraintes réciproquement selon la condition de préhension et/ou de libération de l'extrémité en forme de pince (31a).

9. Dispositif (10) selon l'une quelconque des revendications précédentes, dans lequel ladite cavité interne (23) comporte une partie de guidage (24) configurée pour favoriser/desserrer le serrage de ladite extrémité en forme de pince (31a), de préférence ladite partie de guidage (24) comportant une première partie (24a) ayant un diamètre interne approximativement égal à un diamètre extérieur (« d ») de ladite première tige (31) en correspondance de ladite extrémité en forme de pince (31a) lorsqu'elle est fermée, et une seconde partie (24b) ayant un diamètre supérieur au diamètre extérieur (« d »).

10. Dispositif (10) selon la revendication 9, dans lequel lesdits moyens de génération (30) comprennent un premier élément élastique (50) configuré pour générer une force de poussée sur ladite première tige (31) de sorte à réaliser une course d'engagement (A) à travers ladite partie de guidage (24) vers ladite seconde tige (32), pour permettre ainsi la mise en prise entre ladite extrémité en forme de pince (31a) et ladite extrémité arrondie (32a) dans une configuration fonctionnelle d'utilisation dudit dispositif (10), ladite course d'engagement étant de préférence dirigée de ladite seconde extrémité (22) vers ladite première extrémité (21) dudit corps principal (20).

11. Dispositif (10) selon la revendication 10, dans lequel lesdits moyens de génération (30) comprennent un système d'entraînement (70) associé à ladite première tige (31) et configuré pour exercer une force d'opposition excédant ladite force de poussée dudit premier élément élastique (50) de sorte que ladite première tige (31) et ladite seconde tige (32), dans une condition d'engagement mutuel, effectuent une course de libération (B) via ladite partie de guidage (24) dans une configuration fonctionnelle d'utilisation dudit dispositif (10), ladite course de libération (B) étant opposée à la direction de ladite course d'engagement (A).

12. Dispositif (10) selon la revendication 11, dans lequel ladite seconde partie (24b) de ladite partie de guidage (24) de ladite cavité interne (23) est configurée de manière à permettre le desserrage du serrage de ladite extrémité en forme de pince (31a) sur ladite extrémité arrondie (32a) de ladite seconde tige (32) pour permettre ainsi l'extraction de ladite extrémité arrondie (32a) de ladite partie en forme de pince (31a) dans une condition fonctionnelle de libération desdits moyens de mise en prise et de libération.

13. Dispositif (10) selon l'une ou plusieurs des revendications précédentes, dans lequel lesdits moyens de génération (30) comprennent un second élément élastique (60) configuré pour exercer une force d'impulsion sur ledit élément de butée (40) de manière à le pousser vers ladite première extrémité (21) durant une course d'impulsion (C) desdits moyens de mise en prise et de libération.

14. Dispositif (10) selon l'une quelconque des revendications précédentes, dans lequel ladite force de poussée dudit premier élément élastique (50) est réglable au moyen d'un élément de réglage (80) de préférence disposé en correspondance de ladite seconde extrémité (22) dudit corps principal (20).

15. Pistolet à clouer (300) destiné aux applications d'implant osseux comprenant un dispositif servant à générer une force d'impulsion (10) selon l'une ou plusieurs des revendications précédentes et une tête pour maintenir un clou (400), de préférence ladite tête servant à maintenir un clou (400) étant fixée à la première extrémité (21) du corps principal (20) dudit dispositif de génération de force (10).

16. Pistolet à clouer (300) selon la revendication 15, dans lequel ladite tête servant à maintenir un clou (400) inclut un clou (100) comportant une partie de tête (110) orientée vers ledit élément de butée (40) dudit dispositif (10) dans une configuration fonctionnelle d'utilisation dudit pistolet à clouer (400).
